# FASCICULE DE BREVET EUROPEEN

(11) **EP 0 787 131 B1**
(45) Date de publication et mention de la délivrance du brevet: **31.10.2001**
(21) Numéro de dépôt: 96928492.6
(22) Date de dépôt: 07.08.1996
(51) Int. Cl.: C07D 401/12, A61K 31/47, C07D 409/12, C07D 215/26

(54) **DERIVES DE BENZENESULFONAMIDE COMME ANTAGONISTES DE LA BRADYKININE**
BENZENSULFONAMIDDERIVATE ALS BRADYKININ ANTAGONISTEN
BENZENESULPHONAMIDE DERIVATIVES AS BRADYKININE ANTAGONISTS

(30) Priorité: 17.08.1995 FR 9509885
(43) Date de publication de la demande: 06.08.1997
(73) Titulaire: FOURNIER INDUSTRIE ET SANTE, F-75008 Paris (FR)
(72) Inventeur: DODEY, Pierre, F-21121 Fontaine-les-Dijon (FR); BONDOUX, Michel, F-21121 Fontaine-les-Dijon (FR); HOUZIAUX, Patrick, F-78580 Bazemont (FR); BARTH, Martine, F-78490 Montfort-l'Amaury (FR); OU, Khan, F-21121 Hauteville-Les-Dijon (FR)
(74) Mandataire: Hubert, Philippe
(86) Numéro de dépôt international: FR9601262
(87) Numéro de publication internationale: WO9707115

(56) Documents cités:
- EP-A- 0 596 406
- EP-A- 0 622 361

## Description

### Domaine de l'invention

La présente invention concerne de nouveaux composés de benzènesulfonamide, leur procédé de préparation et leur utilisation en thérapeutique.

Ces nouveaux composés présentent notamment une action antagoniste vis-à-vis de la bradykinine et sont utiles en thérapeutique, particulièrement pour le traitement de la douleur, de l'inflammation, de l'asthme et des rhinites allergiques.

### Art antérieur

On sait que l'une des possibilités de traitement de certaines pathologies à caractère douloureux et/ou inflammatoire (telles que l'asthme, la rhinite, le choc septique, la douleur dentaire, etc...) est d'antagoniser l'action de certaines hormones telles que la bradykinine ou la kallidine. En effet ces hormones peptidiques sont impliquées dans un grand nombre de processus physiologiques dont certains sont liés de façon étroite à ces pathologies.

Bien qu'actuellement aucun produit possédant ce mode d'action ne soit encore commercialisé, de nombreuses études ont été entreprises pour créer des composés susceptibles d'être antagonistes de récepteurs de la bradykinine. La bradykinine est une hormone peptidique constituée de 9 aminoacides (Arg-Pro-Pro-Gly-Phe-Ser-Pro-Phe-Arg) et la kallidine est une hormone peptidique (Lys-Arg-Pro-Pro-Gly-Phe-Ser-Pro-Phe-Arg) qui comporte un aminoacide supplémentaire (Lys) par rapport à la bradykinine. On sait que des études antérieures ont permis d'obtenir des peptides qui réagissent avec les récepteurs de la bradykinine : certains comme le Bradycor (CP.0127 de la société Cortech), l'Icatibant (HOE 140 de la société Hoechst) ["Bradycor" et "Icatibant" sont des dénominations communes internationales (DCI)] ou encore le NPC 17761 (de la société Scios-Nova) présentent une action inhibitrice de la fixation de la bradykinine sur le récepteur B₂ de la bradykinine. Plus récemment, des composés non peptidiques ont été proposés comme antagonistes de la bradykinine vis-à-vis de la fixation sur le récepteur B₂ de la bradykinine, notamment dans EP-A-0596406 et EP-A-0622361. On sait en outre que certains composés de structure apparentée à celles des composés visés dans les deux demandes de brevet précitées ont déjà été décrits, notamment dans DE-A-3617183 et dans EP-A-0261539, eu égard à leurs éventuelles propriétés antithrombotiques.

### But de l'invention

Il existe un besoin d'atténuer ou de supprimer chez le mammifère et surtout chez l'homme les douleurs et les inflammations.

Pour satisfaire ce besoin, on a recherché une nouvelle solution technique qui soit efficace dans le traitement des algies quelle que soit leur origine, notamment celles liées à des phénomènes inflammatoires, d'une part, et dans le traitement des inflammations, d'autre part.

Selon l'invention, on se propose de fournir une nouvelle solution technique, qui met en oeuvre une fixation compétitive au niveau du récepteur B₂ de la bradykinine entre (i) la bradykinine et les hormones apparentées ou analogues, et (ii) une substance antagoniste, et qui fait appel à des composés de benzènesulfonamide structurellement différents des produits connus précités et qui limitent ou inhibent substantiellement la fixation de la bradykinine et des hormones analogues sur ledit récepteur B₂ de la bradykinine.

Conformément à cette nouvelle solution technique on se propose de fournir, selon un premier aspect de l'invention, des composés de benzènesulfonamide en tant que produits industriels nouveaux ; selon un second aspect de l'invention, un procédé de préparation de ces composés ; et selon un troisième aspect de l'invention, l'utilisation de ces composés notamment en thérapeutique en tant qu'ingrédients actifs antalgiques et/ou anti-inflammatoires.

### Objet de l'invention

Selon la nouvelle solution technique de l'invention, on préconise en tant que produit industriel nouveau, un composé de benzènesulfonamide qui est caractérisé en ce qu'il est choisi parmi l'ensemble constitué par :
**(i)** les composés de formule : 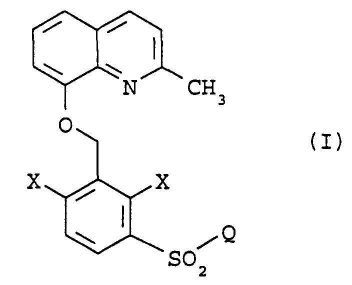 dans laquelle :
   - X: représente un atome d'halogène,
   - Q: représente un reste N-hétérocyclique de structure : 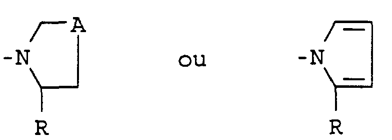
   - A: représente un groupe -CH₂-, -CH(OH)-, -CH(NH-COCH₃)- ou un atome de soufre,
   - R: représente un atome d'hydrogène, un groupe CO₂H, CO₂-B-R₁ ou CO-N(R₂)-B-R₁,
   - B: représente un groupe alkylène en C₁-C₁₀, linéaire, ramifié ou cyclisé,
   - R₁: représente un atome d'hydrogène, un groupe CH₂OH, CH₂-O-CH₃, CH₂-NR₃R₄ ou phényle,
   - R₂: représente un atome d'hydrogène ou un groupe alkyle en C₁-C₄,
   - R₃: représente un atome d'hydrogène ou un groupe alkyle en C₁-C₁₀, linéaire, ramifié ou cyclisé,
   - R₄: représente un atome d'hydrogène ou un groupe alkyle en C₁-C₁₀ linéaire ou ramifié,
   - NR₃R₄: pouvant représenter un reste hétérocyclique saturé ayant de 5 à 8 sommets et comportant au moins un atome d'azote,
   le carbone porteur du substituant R, lorsque Q est saturé, peut être de configuration non déterminée (R,S) ou de configuration déterminée (R), ou (S) ; et,
**(ii)** leurs sels d'addition.
   Selon l'invention, on préconise aussi un procédé de préparation des composés de formule I et de leurs sels d'addition.
   On préconise également l'utilisation d'une substance choisie parmi les composés de formule I et leurs sels d'addition non-toxiques pour l'obtention d'un médicament destiné à une utilisation en thérapeutique vis-à-vis des pathologies impliquant la bradykinine ou ses analogues, en particulier vis-à-vis des algies, et notamment dans le traitement ou la prévention de pathologies liées à des états inflammatoires ou douloureux.

### Description détaillée de l'invention

Dans la formule générale I des composés de l'invention, on entend par atome d'halogène un atome de fluor, de chlore, de brome ou d'iode, l'halogène préféré étant l'atome de chlore en ce qui concerne l'activité pharmacologique ou thérapeutique.

Par groupes alkyle en C₁-C₄, on entend ici les groupes méthyle, éthyle, propyle, 1-méthyléthyle, butyle, 1,1-diméthyléthyle, 1-méthylpropyle et 2-méthylpropyle. Par groupes alkylène en C₁-C₁₀, linéaire, ramifié ou cyclisé, on entend les chaînes hydrocarbonées saturées linéaires comportant entre 1 et 10 atomes de carbone, les chaînes hydrocarbonées saturées ramifiées comportant 3 à 10 atomes de carbone et les chaînes hydrocarbonées comportant dans leur structure au moins un cycle saturé formé par 3 à 6 atomes de carbone.

Dans la formule I, le reste hétérocyclique Q peut être saturé : 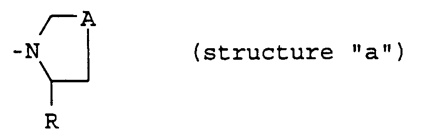 ou insaturé :

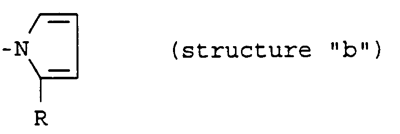

Par reste N-hétérocyclique comportant au moins un atome d'azote, on entend une structure cyclique saturée de 5 à 8 sommets (i.e. formée de 5 à 8 atomes), l'un au moins des atomes de ce cycle étant l'atome d'azote. En pratique le cycle NR₃R₄ peut comporter un second hétéroatome choisi parmi O, N et S et peut être substitué notamment par un groupe alkyle en C₁-C₄, ω-hydroxyalkyle en C₁-C₄, phényle, 4-méthylphényle ou 4-halogénophényle. Parmi les groupes N-hétérocycliques NR₃R₄ utilisables selon l'invention, on peut mentionner en particulier les groupes pyrrolidino, pipéridino, 4-méthylpipéridino, morpholino, pipérazino, 4-méthylpipérazin-1-yle, 4-(2-hydroxyéthyl)pipérazin-1-yle, 4-phénylpipérazin-1-yle, 4-(4-chlorophényl)pipérazin-1-yle, thiazolidino (ou 3-thiazolidinyle), oxazolidino (ou 3-oxazolidinyle) et hexaméthylèneimino. Les groupes N-hétérocycliques NR₃R₄ préférés selon l'invention sont les groupes pyrrolidino, pipéridino, 4-méthylpipérazin-1-yle, 4-phénylpipérazin-1-yle, thiazolidino, oxazolidino et hexaméthylèneimino.

Par "sels d'addition", on entend les sels d'addition d'acide obtenus par réaction d'un composé de formule I avec un acide minéral ou un acide organique. Les acides minéraux préférés pour salifier un composé basique de formule I sont les acides chlorhydrique, bromhydrique, phosphorique et sulfurique. Les acides organiques préférés pour salifier un composé basique de formule I sont les acides méthanesulfonique, maléique, fumarique, oxalique, citrique et trifluoroacétique.

Le procédé de préparation des composés de formule I où Q est un reste cyclique saturé que l'on préconise selon l'invention comprend, selon une première variante A, les étapes consistant à :
**1)** faire réagir un composé de formule : 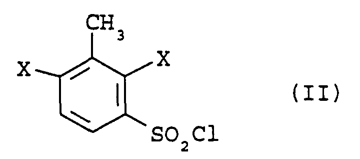 dans laquelle X représente un atome d'halogène,
   avec un agent d'halogénation tel que par exemple le N-bromosuccinimide ou le N-chlorosuccinimide, dans un solvant, par exemple un hydrocarbure halogéné, à une température proche de la température de reflux du solvant, en présence d'un initiateur radicalaire comme par exemple du peroxyde de benzoyle, pendant 0,5 à 5 heures, pour obtenir un composé de formule : 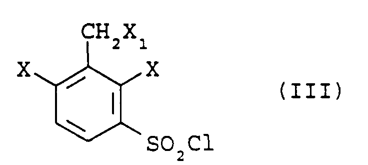 dans laquelle X et X₁ représentent chacun un halogène ;
**2)** condenser le composé de formule III ainsi obtenu avec un dérivé hétérocyclique de formule : 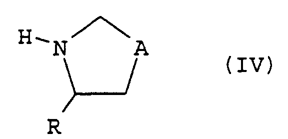 dans laquelle :
   A représente un groupe -CH₂-, -CH(OH)-, -CH(NH-COCH₃)-, ou un atome de soufre,
   R représente un atome d'hydrogène, un groupe COOCH₃ ou un groupe CONHRₐ, où Rₐ représente un groupe alkyle en C₁-C₃, le carbone porteur du substituant R étant de configuration (R,S), (R) ou (S)
   dans un solvant tel que par exemple du dichlorométhane, en présence d'une base comme par exemple la triéthylamine, à une température comprise entre 0 et 40° C, pendant 0,5 à 3 heures, pour obtenir un composé de formule : 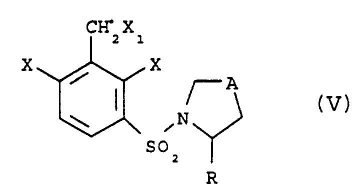 dans laquelle X, A et R conservent la même signification que dans les composés de départ ;
**3)** faire réagir le composé de formule V obtenu ci-dessus avec un composé de formule : 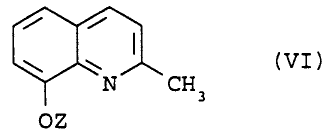 dans lequel Z représente un métal alcalin tel que le sodium ou le potassium,
   dans un solvant anhydre, comme par exemple le diméthylformamide ou le tétrahydrofurane, à température comprise entre 0 et 50° C, pendant 0,5 à 5 heures, pour obtenir un composé de formule : 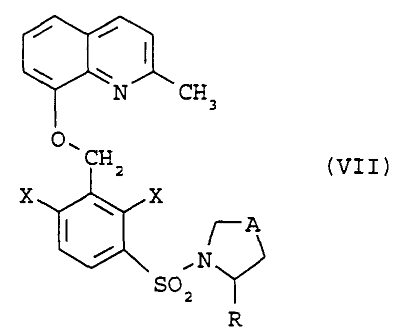 dans laquelle X, A et R conservent la même signification que dans les composés de départ, et le carbone porteur du substituant R conserve la même configuration que dans le composé IV de départ ;
**4)** si nécessaire, lorsque dans le composé de formule VII ci-dessus R représente un groupe ester, effectuer une hydrolyse alcaline de la liaison ester, par action d'une solution aqueuse d'hydroxyde de sodium dans un solvant comme par exemple du diméthoxyéthane, à une température comprise entre 10 et 50° C, pendant 1 à 30 heures pour obtenir, après acidification, le composé acide de formule : 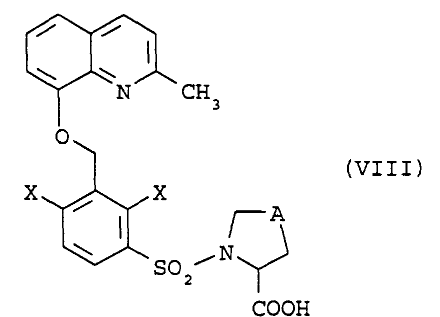 dans laquelle X et A conservent la même signification que dans le composé VII de départ ;
**5)** si nécessaire, faire réagir l'acide VIII ainsi obtenu avec un alcool ou une amine de formules respectives

   HO-B-R₁ (IX) ou HN(R₂)-B-R₁ (IX')

   dans lesquelles :
   B représente une chaîne alkylène en C₁-C₁₀, linéaire, ramifiée ou comportant un cycle saturé,
   R₁ représente
      un atome d'hydrogène,
      un groupe -CH₂-O-CH₃.
      un groupe -CH₂-N(CH₃)₂ ou
      un groupe phényle,
   R₂ représente un atome d'hydrogène ou un groupe alkyle en C₁-C₄, dans un solvant, comme par exemple le dichlorométhane en présence d'activateurs, comme par exemple, le 1-hydroxy-7-aza-benzotriazole (HOAT) et le chlorhydrate de 1-[3-(diméthylaminopropyl)-3-éthyl]carbodiimide (EDCI) à température proche de la température ambiante, pendant 2 à 50 heures, pour obtenir un composé de formule : 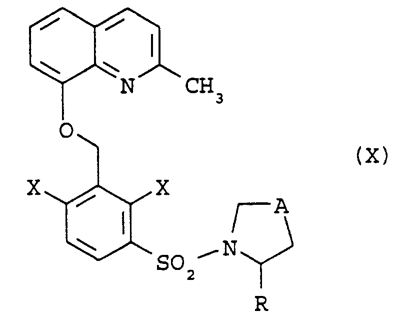
   dans laquelle
   X et A conservent la même signification que dans les composés de départ et
   R représente un groupe COO-B-R₁ ou CO-N(R₂)-B-R₁, où B, R₁ et R₂ conservent la même signification que dans les composés IX ou IX' de départ, et le carbone portant le substituant R conserve la même configuration que dans le composé IV de départ.

Selon une seconde variante B du procédé de préparation d'un composé de formule I, où Q est le reste pyrrolidino, on effectue la réaction d'un composé de formule : 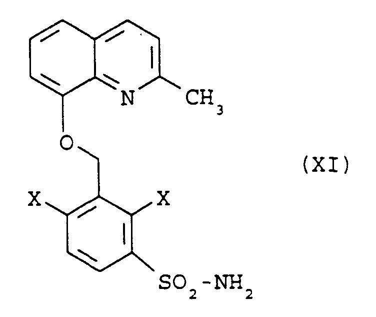 dans laquelle X représente un halogène,
avec du 1,4-dibromobutane, en présence d'une base comme par exemple du carbonate de potassium, dans un solvant, comme par exemple l'acétonitrile, à une température comprise entre 30° C et la température de reflux du solvant, pendant 3 à 30 heures, pour obtenir un composé de formule :

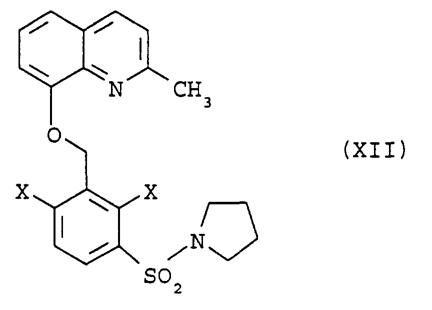

### Meilleur mode

Le meilleur mode de mise en oeuvre de l'invention consiste à faire appel à des composés de formule I et leurs sels d'addition où
- X: représente un atome de chlore,
- R: représente un groupe CO₂-B-R₁ ou CONH-B-R₁, dans lesquels R₁ est CH₂-NR₃R₄, et B est un groupe alkylène en C₁-C₁₀ à chaîne hydrocarbonée linéaire et saturée,
le cycle Q est saturé, et le carbone porteur du substituant R (différent de H) est de configuration déterminée de formule : 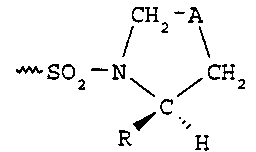 (cette structure correspond à une configuration chirale (S) lorsque A est un groupe CH₂ et à une configuration (R) lorsque A est un atome de soufre). Ces composés sont les préférés selon l'invention.

L'invention sera mieux comprise à la lecture des exemples de préparation qui suivent et des résultats d'essais pharmacologiques obtenus avec certains composés selon l'invention. Dans le cas de composés présentant dans leur structure un carbone asymétrique, l'absence d'indication particulière, ou la mention (R,S) [ou (D,L) dans le cas d'acides aminés] signifie qu'il s'agit de composés racémiques; dans le cas de composés présentant une chiralité, celle-ci est indiquée à la suite immédiate de l'indexation du substituant porté par ledit carbone asymétrique; on utilise alors les signes (R) ou (S), conformément aux règles de Cahn, Ingold et Prelog, ou, dans le cas d'acides aminés les mentions (D) ou (L). La nomenclature utilisée dans les exemples est celle préconisée par les Chemical Abstracts : ainsi certains dérivés de la L-proline peuvent devenir, après réaction de la fonction acide avec une amine, des dérivés de la 2-(S)-pyrrolidinecarboxamide.

Dans la partie expérimentale, les "préparations" sont relatives aux composés intermédiaires et les "exemples" sont relatifs aux produits selon l'invention.

Certains composés sont caractérisés par les données spectrales obtenues en résonance magnétique nucléaire (RMN) ; dans ce cas, les caractéristiques spectrales sont données pour le proton (¹H), et on indique le déplacement chimique des protons par rapport au signal proton du tétraméthylsilane avec, entre parenthèses, la forme du signal (s pour singulet, d pour doublet, t pour triplet, q pour quadruplet, m pour multiplet, sl pour signal large) et le nombre de protons pour le signal. A titre indicatif, les spectres RMN¹H ont été réalisés à 250 MHz.

Les points de fusion (F) indiqués ci-après sont en général mesurés à l'aide d'un banc Koffler et ne sont pas corrigés, il s'agit alors de points de fusion instantanée.

### PREPARATION I

### Chlorure de 3-bromométhyl-2,4-dichlorobenzènesulfonyle

A une solution à température ambiante de 41,52 g (0,16 mole) de chlorure de 2,4-dichloro-3-méthylbenzènesulfonyle dans 150 ml de 1,1,2,2-tétrachloroéthane on ajoute 85,44 g (0,48 mole) de N-bromosuccinimide puis 200 mg de peroxyde de benzoyle. Le mélange réactionnel est porté à 120° C pendant 2 heures. Après avoir refroidi, on filtre et le filtrat est lavé successivement à l'eau par une solution saturée de bicarbonate de sodium et enfin à l'eau, jusqu'à neutralité. Puis la phase organique est séchée sur sulfate de magnésium et concentrée. Le produit obtenu est cristallisé dans l'hexane. On obtient ainsi 25,53 g du produit attendu sous forme de cristaux blancs (Rendement = 47 %).
**F = 90-92° C**

### PREPARATION II

### Chlorure de 3-chlorométhyl-2,4-dichlorobenzènesulfonyle

A une solution de 6,5 g (0,025 mole) de chlorure de 2,4-dichloro-3-méthylbenzènesulfonyle dans 30 ml de 1,1,2,2-tétrachloroéthane, à température ambiante et sous atmosphère d'azote, on ajoute 10 g (0,075 mole) de N-chlorosuccinimide et 30 mg de peroxyde de benzoyle. Le mélange réactionnel est porté à 120° C pendant 3 heures, refroidi à température ambiante, versé sur de l'eau, puis extrait avec du dichlorométhane. La phase organique est lavée à l'eau, par une solution saturée de bicarbonate de sodium et enfin à l'eau jusqu'à neutralité. Elle est ensuite séchée sur sulfate de magnésium, et concentrée sous pression réduite. Par recristallisation dans l'hexane, on obtient 0,85 g du produit attendu, sous forme de cristaux blancs (Rendement = 11,5 %).
**F = 68°C**

### PREPARATION III

### N-[(3-bromométhyl-2,4-dichlorophényl)sulfonyl]-(D,L)-proline, méthyl ester

A une solution de 13,24 g (0,08 mole) de chlorhydrate de (D,L)-proline, méthyl ester dans 60 ml de dichlorométhane, on ajoute 27,08 g (0,08 mole) de composé obtenu selon la préparation I. On refroidit à 0°C et additionne goutte à goutte une solution de 23,3 ml (0,16 mole) de triéthylamine dans 20 ml de dichlorométhane. On laisse agiter ensuite à température ambiante pendant 30 minutes. Le mélange réactionnel est versé sur de l'eau et extrait au dichlorométhane. La phase organique est lavée par une solution d'acide chlorhydrique 1N puis à l'eau jusqu'à pH neutre. Elle est enfin séchée et concentrée sous pression réduite. On obtient 34,48 g d'huile utilisée directement dans les étapes ultérieures. Le produit obtenu contient également le composé N-[(3-chlorométhyl-2,4-dichlorophényl)sulfonyl]-(D,L)-proline, méthyl ester provenant d'une réaction secondaire. Ce sous-produit possédant sensiblement la même réactivité que le dérivé bromé, on utilise directement le mélange, sans purification complémentaire.

### Exemple 1

### N-[[3-[(2-méthylquinolin-8-yl)oxyméthyl]-2,4-dichlorophényl]sulfonyl]-D,L-proline, méthyl ester

A une solution de 0,270 g (1,7.10⁻³ mole) de 8-hydroxy-2-méthyl-quinoleïne dans 5 ml de N,N-diméthylformamide (DMF) on ajoute 0,051 g (1,7.10⁻³ mole) d'hydrure de sodium à 80 % en suspension dans l'huile. Après agitation pendant dix minutes à température ambiante on ajoute 0,8 g du composé obtenu selon la préparation III en solution dans 2 ml de DMF. L'agitation est maintenue 2 heures à température ambiante. Le mélange réactionnel est étendu d'eau et extrait à l'acétate d'éthyle. Les phases organiques sont lavées à l'eau, séchées sur sulfate de sodium, filtrées et concentrées sous pression réduite. Le résidu obtenu est purifié par chromatographie sur gel de silice en éluant à l'aide d'un mélange toluène/acétate d'éthyle (80/20, v/v). Le solide récupéré est recristallisé dans 15 ml d'isopropanol pour donner 0,7 g du produit attendu sous forme d'une poudre blanche (rendement = 81 %).
**F = 142° C**

### PREPARATION IV

### N-[(3-bromométhyl-2,4-dichlorophényl)sulfonyl]-L-proline, méthyl ester

En opérant de façon analogue à la préparation III, au départ du chlorhydrate de L-proline, méthyl ester, on obtient le produit attendu sous forme d'une huile. (Le produit obtenu contient l'analogue chloré issu d'une réaction secondaire d'échange entre halogène).

### Exemple 2

### N-[[3-[(2-méthylquinolin-8-yl)oxyméthyl]-2,4-dichlorophényl]sulfonyl]-L-proline, méthyl ester

En opérant de façon analogue à l'exemple 1, au départ du composé obtenu selon la préparation IV, on obtient le produit attendu avec un rendement de 83 % après recristallisation dans l'isopropanol.
**F = 136°C**
**[α]**_{**D**}^{**23**} **= +29,4° (c = 1,01 ; CHCl**_{**3**}**)**

### Exemple 3

### N-[[3-[(2-méthylquinolin-8-yl)oxyméthyl]-2,4-dichlorophényl]sulfonyl]-D,L-proline

A une solution de 5,14 g (10,2.10⁻³ mole) du composé obtenu selon l'exemple 1, dans 100 ml de 1,2-diméthoxyéthane, on ajoute 20 ml (20.10⁻³ mole) d'une solution aqueuse d'hydroxyde de sodium 1N. Le mélange réactionnel est agité à 40° C pendant 1,5 heure, puis à température ambiante pendant 20 heures. Le mélange est ensuite concentré sous pression réduite et le résidu est repris à l'eau et acidifié jusqu'à pH5 à l'aide d'acide chlorhydrique 1N. Après extraction à l'aide de dichlorométhane, la phase organique est lavée à l'eau, séchée et concentrée sous pression réduite. Le solide ainsi obtenu est recristallisé dans 30 ml d'isopropanol pour donner 4,4 g du produit attendu sous forme de cristaux blancs (rendement = 87 %).
**F = 120°C**

### Exemple 4

### 1-[[3-[(2-méthylquinolin-8-yl)oxyméthyl]-2,4-dichlorophényl]sulfonyl]-N-[2-méthoxyéthyl]-2-pyrrolidinecarboxamide

On prépare une solution de 0,495 g (10⁻³ mole) de l'acide obtenu selon l'exemple 3, dans 5 ml de dichlorométhane et on ajoute 0,21 g (1,1.10⁻³ mole) de chlorhydrate de 1-(3-diméthylaminopropyl)-3-éthyl-carbodiimide (EDCI), 0,15 g (1,1.10⁻³ mole) de 1-hydroxy-7-azabenzotriazole (HOAT), puis 0,15 g (2.10⁻³ mole) de 2-méthoxyéthanamine. Le mélange réactionnel est agité à température ambiante (20-25° C) pendant 20 heures puis concentré sous pression réduite. Le résidu obtenu est purifié par chromatographie sur gel de silice en éluant avec un mélange toluène/isopropanol (9/1, v/v). Après recristallisation dans un mélange toluène/éther isopropylique (1/8, v/v), on obtient 0,34 g du produit attendu sous forme de cristaux blancs. (Rendement = 61 %).
**F = 107°C**

### Exemple 5

### 1-[[3-[(2-méthylquinolin-8-yl)oxyméthyl]-2,4-dichlorophényl]sulfonyl]-N-[2-hydroxyéthyl]-2-pyrrolidinecarboxamide

En opérant de façon analogue au procédé de l'exemple 4, au départ de 2-aminoéthanol, on obtient après recristallisation dans l'acétate d'éthyle, le produit attendu avec un rendement de 85 %.
**F = 197°C**

### Exemple 6

### 1-[[3-[(2-méthylquinolin-8-yl)oxyméthyl]-2,4-dichlorophényl]sulfonyl]-N-[3-hydroxypropyl]-2-pyrrolidinecarboxamide

En opérant de façon analogue au procédé de l'exemple 4, au départ de 3-aminopropanol, on obtient après recristallisation dans l'acétate d'éthyle, le produit attendu avec un rendement de 61 %.
**F = 147°C**

### Exemple 7

### 1-[[3-[(2-méthylquinolin-8-yl)oxyméthyl]-2,4-dichlorophényl]sulfonyl]-N-[5-hydroxypentyl]-2-pyrrolidinecarboxamide

En opérant de façon analogue au procédé de l'exemple 4, au départ de 5-amino-pentanol, on obtient après recristallisation dans un mélange acétate d'éthyle/éther isopropylique, le produit attendu avec un rendement de 74 %.
**F = 64°C**

### Exemple 8

### Dichlorhydrate de 1-[[3-((2-méthylquinolin-8-yl)oxyméthyl]-2,4-dichlorophényl]sulfonyl]-N-[2-diméthylaminoéthyl]-2-pyrrolidinecarboxamide

En opérant de façon analogue au procédé de l'exemple 4, au départ de N,N-diméthyléthylènediamine, on obtient le composé attendu sous forme de base. Celui-ci est ensuite repris en solution dans l'acétate d'éthyle, puis on ajoute une solution de chlorure d'hydrogène dans l'éther éthylique. Le dichlorhydrate attendu cristallise et on le sépare par filtration (rendement = 70 %).
**F = 151°C**

### PREPARATION V

### 1-[[3-[(2-méthylquinolin-8-yl)oxyméthyl]-2,4-dichlorophényl]sulfonyl]-N-[3-[(1,1-diméthyléthoxycarbonyl)amino]propyl]-2-pyrrolidinecarboxamide

En opérant de façon analogue au procédé de l'exemple 4, au départ de 3-[(1,1-diméthyléthoxycarbonyl)amino]-propanamine, on obtient le produit attendu sous forme d'un solide blanc avec un rendement de 53 %.
**F = 88°C**

### Exemple 9

### 1-[[3-[(2-méthylquinolin-8-yl)oxyméthyl]-2,4-dichlorophényl]sulfonyl]-N-[3-aminopropyl]-2-pyrrolidinecarboxamide

On prépare un mélange de 0,7 g (1,1.10⁻³ mole) du composé obtenu selon la préparation V et de 0,12 g (1,1.10⁻³ mole) d'anisol, puis on ajoute 1,7 ml d'acide trifluoroacétique. La solution obtenue est agitée pendant 3 heures à température ambiante puis concentrée sous pression réduite. Le résidu obtenu est amené à neutralité à l'aide d'une solution d'hydroxyde de sodium 1N et le produit est extrait à l'aide de dichlorométhane. La phase organique est lavée à l'eau, séchée et concentrée. Le produit brut est purifié par chromatographie sur gel de silice en éluant avec un mélange dichlorométhane/méthanol (8/2, v/v). On obtient ainsi 40 mg du produit attendu sous forme d'un solide beige (rendement = 7,7 %).
**F = 130-132 °C**

### Exemple 10

### 1-[[3-[(2-méthylquinolin-8-yl)oxyméthyl]-2,4-dichlorophényl]sulfonyl]-N-[phénylméthyl]-2-pyrrolidinecarboxamide

En opérant de façon analogue au procédé de l'exemple 4, au départ de benzylamine, on obtient le produit attendu avec un rendement de 86 % après recristallisation dans l'éther isopropylique.
**F = 143°C**

### Exemple 11

### N-[[3-[(2-méthylquinolin-8-yl)oxyméthyl]-2,4-dichlorophényl]sulfonyl]-D,L-proline, 3-(diméthylamino)propyl ester

En opérant de façon analogue au procédé de l'exemple 4, au départ de 3-(diméthylamino)propanol, on obtient le produit attendu avec un rendement de 57 % après recristallisation dans l'éther isopropylique.
**F = 93°C**

### PREPARATION VI

### 1-[[3-(bromométhyl)-2,4-dichlorophényl]sulfonyl]-N-éthyl-2-(S)-pyrrolidinecarboxamide

En opérant de façon analogue au procédé de la préparation III, au départ N-éthyl-2-(S)-pyrrolidinecarboxamide, on obtient le produit attendu, utilisé directement dans l'étape suivante.

### Exemple 12

### 1-[[3-[(2-méthylquinolin-8-yl)oxyméthyl]-2,4-dichlorophényl]sulfonyl]-N-éthyl-2-(S)-pyrrolidinecarboxamide

En opérant de façon analogue au procédé de l'exemple 1, au départ du composé obtenu selon la préparation VI, on obtient le produit attendu avec un rendement de 72 % après recristallisation dans un mélange acétate d'éthyle/éther isopropylique.
**F = 80°C**
**[α]**_{**D**}^{**25**} **= -33,8° (c = 1,02 ; CH**_{**3**}**OH)**

### PREPARATION VII

### 3-[(1-oxoéthyl)amino]-1-[[3-(bromométhyl)-2,4-dichlorophényl]sulfonyl]pyrrolidine

En opérant de façon analogue au procédé de la préparation III, au départ de 3-(acétylamino)pyrrolidine, on obtient le produit attendu qui est utilisé directement au stade suivant.

### Exemple 13

### 3-[(1-oxoéthyl)amino]-1-[[3-[(2-méthylquinolin-8-yl)oxyméthyl]-2,4-dichlorophényl]sulfonyl]pyrrolidine

En opérant de façon analogue au procédé de l'exemple 1, au départ du composé obtenu selon la préparation VII, on obtient le produit attendu avec un rendement de 48 %.
**F = 142°C**

### PREPARATION VIII

### 3-hydroxy-1-[[3-(bromométhyl)-2,4-dichlorophényl]sulfonyl]pyrrolidine

A une solution de 1 g (2,95.10⁻³ mole) du composé obtenu selon la préparation I, dans 20 ml de dichlorométhane, on ajoute 0,32 g (2,95.10⁻³ mole) de lutidine et 0,26 g (2,95.10⁻³ mole) de 3-pyrrolidinol. Le mélange est agité pendant 3 heures à température ambiante, puis versé sur 50 ml d'acide chlorhydrique 1N et extrait à l'aide de dichlorométhane. La phase organique est lavée à l'eau puis séchée et concentrée. Le résidu est purifié par chromatographie sur gel de silice en éluant avec un mélange toluène/acétate d'éthyle (1/1, v/v). On obtient ainsi 0,6 g du produit attendu. Celui-ci contient son analogue chlorométhylé en raison d'une réaction d'échange des halogènes. Le produit obtenu ainsi est utilisé directement pour le stade suivant.

### Exemple 14

### 3-hydroxy-1-[[3-[(2-méthylquinolin-8-yl)oxyméthyl]-2,4-dichlorophényl]sulfonyl]pyrrolidine

En opérant de façon analogue au procédé de l'exemple 1, au départ du composé obtenu selon la préparation VIII, on obtient le produit attendu sous forme de cristaux blancs avec un rendement de 76 %.

### PREPARATION IX

### 3-[[3-(bromométhyl)-2,4-dichlorophényl]sulfonyl]thiazolidine

En opérant de façon analogue au procédé la préparation III, au départ de thiazolidine, on obtient le produit attendu qui est utilisé au stade suivant sans purification complémentaire.

### Exemple 15

### 3-[[3-[(2-méthylquinolin-8-yl)oxyméthyl]-2,4-dichlorophényl]sulfonyl]thiazolidine

En opérant de façon analogue au procédé de l'exemple 1, au départ du composé obtenu selon la préparation IX, on obtient le produit attendu avec un rendement de 39 %.
**F = 130°C**

### Exemple 16

### 1-[[3-[(2-méthylquinolin-8-yl)oxyméthyl]-2,4-dichlorophényl]sulfonyl]pyrrolidine

On prépare une solution de 0,5 g (0,98.10⁻³ mole) de 3-[(2- méthylquinolin-8-yloxy)méthyl]-2,4-dichlorobenzènesulfonamide dans 50 ml d'acétonitrile et on ajoute 0,5 g (3,9.10⁻³ mole) de carbonate de potassium et 0,2 g (0,98.10⁻³ mole) de 1,4-dibromobutane. On porte le mélange réactionnel à reflux du solvant pendant 20 heures, puis on filtre. Le filtrat est concentré sous pression réduite, le résidu est repris à l'aide d'une solution diluée de bicarbonate de sodium, et extrait par du dichlorométhane. La phase organique est lavée à l'eau, séchée et concentrée. Après lavage du résidu solide à l'aide d'éther éthylique, on obtient 0,25 g du produit attendu sous forme d'un solide beige (rendement = 62 %).
**F = 204°C**

### PREPARATION X

### 1-[[3-(bromométhyl)-2,4-dichlorophényl]sulfonyl]pyrrole

On prépare une solution de 0,162 g (2,4.10⁻³ mole) de pyrrole dans 2 ml de tétrahydrofurane (THF) et on ajoute 0,097 g (2,4.10⁻³ mole) de potassium. On chauffe doucement le milieu réactionnel jusqu'au reflux du solvant puis on refroidit à température ambiante et on ajoute une solution de 0,82 g du composé obtenu selon la préparation I dans 2 ml de THF. On maintient ensuite sous agitation pendant 12 heures à température ambiante puis on filtre le milieu réactionnel. Le filtrat est ensuite concentré et le résidu obtenu est purifié par chromatographie sur gel de silice en éluant par du toluène. On obtient ainsi 0,190 g du produit attendu sous forme d'un solide pâteux (rendement = 21,3 %).
RMN¹H (CDCl₃) : 4,73 (s,2H) ; 6,35 (d,2H) ; 7,21 (d,2H) ; 7,45 (d,1H) ; 7,65 (d,1H).

### Exemple 17

### 1-[[3-[(2-méthylquinolin-8-yl)oxyméthyl]-2,4-dichlorophényl]sulfonyl]pyrrole

En opérant de façon analogue au procédé de l'exemple 1, au départ du composé obtenu selon la préparation X, on obtient le produit attendu avec un rendement de 36 %, sous forme de cristaux blancs.
**F = 216-217°C**

L'activité de certains des produits selon l'invention a été évaluée en fonction de leur aptitude à se lier aux récepteurs de la bradykinine. En effet, les kinines, dont le principal représentant est la bradykinine, forment un groupe de petits peptides qui contribuent de façon importante à la réponse inflammatoire et apparaissent de ce fait impliqués dans la pathophysiologie des maladies inflammatoires. De plus, la bradykinine est un des agents algésiants parmi les plus puissants connus. Les kinines activent deux types de récepteurs appelés respectivement B₁ et B₂. Le récepteur B₂ appartient à la grande famille des récepteurs à sept domaines transmembranaires couplés aux G-protéines. Nous décrivons dans la présente invention des composés se liant au récepteur B₂ et bloquant de ce fait la fixation de la bradykinine.

Le test pharmacologique utilisé est le suivant : des segments d'iléon de cobayes mâles de souche Dunkin-Hartley (Iffa Credo, l'Arbresle, France) sont isolés et broyés dans le tampon TES suivant : TES 25mM, 1,10-phénanthroline 1mM (pH 6.8), bacitracine 140 *µ*g/ml, BSA 1g/l. Les membranes sont ensuite isolées par centrifugation (18000 tours par minute ; 20 min ; 4° C). Les études de liaison sont effectuées dans le tampon TES en utilisant la [³H]-bradykinine (120 pM), 50 *µ*g de protéine membranaire par essai (volume final 500 *µ*l) avec un temps d'équilibre de 90 min à 20°C. On détermine ensuite le taux (en pourcentage) d'inhibition de la fixation de [³H]-bradykinine en présence de l'un des composés selon l'invention à tester à une concentration de 10⁻⁶ M.

Les résultats obtenus (notés "activité") lors de ces essais sont consignés dans le tableau I ci-après en regard des exemples figurant dans la description.

Les composés de la présente invention qui inhibent la liaison de la [³H] bradykinine au récepteur B₂ de cobaye (voir tableau I) se lient également au récepteur B₂ humain cloné et transfecté de façon stable dans des cellules CHO (Chinese Hamster Ovary Cells). Ainsi dans ce test, certains composés inhibent à la concentration de 10 *µ*M d'au moins 95 % la fixation de la [³H]-bradykinine au récepteur B₂.

Les composés de la présente invention peuvent être utiles dans le traitement des algies, et en particulier dans le traitement de nombreuses pathologies impliquant la bradykinine ou ses homologues. Parmi ces pathologies, on inclut les chocs septiques et hémorragiques, les réactions anaphylactiques, l'arthrose, la polyarthrite rhumatoïde, les rhinites, l'asthme, les maladies inflammatoires du tractus gastrointestinal (par ex. colites, rectites, maladie de Crohn), la pancréatite, certains carcinomes, l'angiooedème héréditaire, la migraine, les accidents vasculaires cérébraux, certains désordres neurologiques, les états inflammatoires vasculaires (par exemple : athérosclérose et artérite des membres inférieurs), les états douloureux (par exemple douleur dentaire, douleurs menstruelles), les contractions utérines prématurées, la cystite et les brûlures.

Les composés de la présente invention, qui peuvent être utilisés sous forme de base libre ou de leurs sels d'addition non-toxiques, en association avec un excipient physiologiquement acceptable, seront en général prescrits en thérapie à des doses d'environ 1 à 1000 mg/jour, sous une forme administrable par voie orale, par injection intraveineuse, intramusculaire ou sous-cutanée, par voie transdermique, par le moyen d'aérosols ou par le moyen de suppositoires.

Les composés pourront également être administrés par voie topique, par exemple sous forme de gels ou de pommades.

Les composés de la présente invention trouvent également leur utilité, en tant que réactifs pharmacologiques, notamment pour l'étude des interactions hormone-récepteur. L'utilisation en tant que réactif pharmacologique peut faire appel à un dérivé radiomarqué de l'un des composés selon l'invention (par exemple avec du tritium [³H] ou du soufre [³⁵S]), dans le but d'obtenir un radio-ligand destiné à des études conformationnelles du récepteur B₂ de la bradykinine, ou des tests de fixation à ce type de récepteur, par exemple pour l'évaluation de nouveaux composés susceptibles de présenter une affinité pour le récepteur B₂ de la bradykinine.

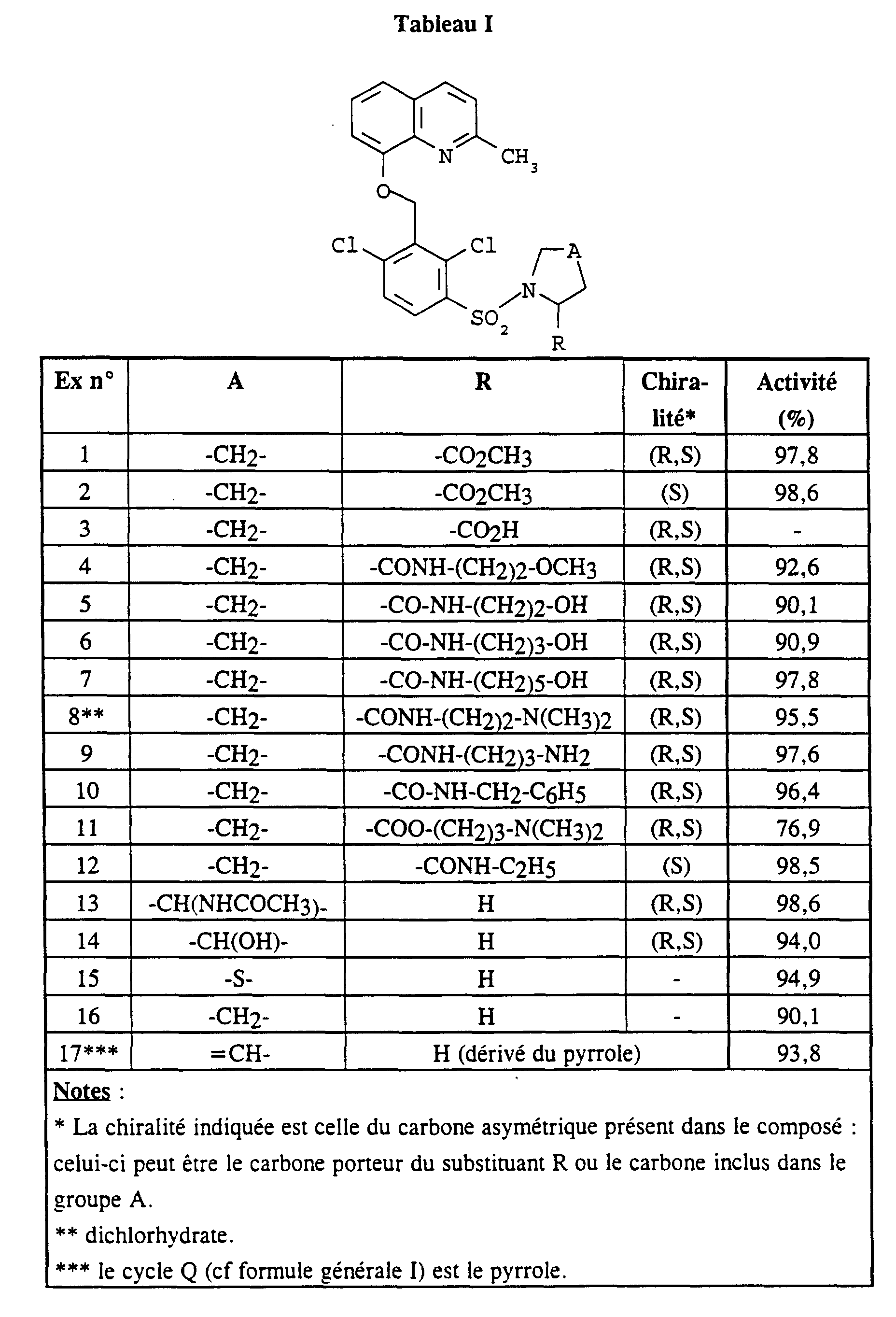

## Revendications

1. Composé de benzènesulfonamide, **caractérisé en ce qu'**il est choisi parmi l'ensemble constitué par :
**(i)** les composés de formule : 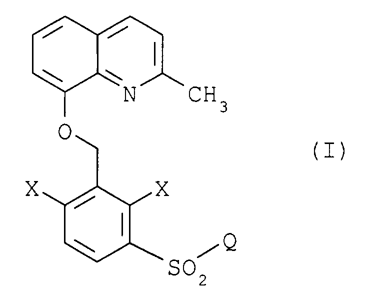 dans laquelle :
X représente un atome d'halogène,
Q représente un reste N-hétérocyclique de structure : 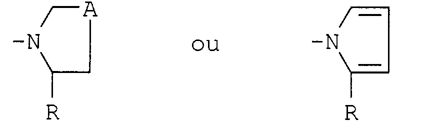
A représente un groupe -CH₂-, -CH(OH)-, -CH(NH-COCH₃)- ou un atome de soufre,
R représente un atome d'hydrogène, un groupe CO₂H, CO₂-B-R₁ ou CO-N(R₂)-B-R₁,
B représente un groupe alkylène en C₁-C₁₀, linéaire, ramifié ou cyclisé,
R₁ représente un atome d'hydrogène, un groupe CH₂OH, CH₂-O-CH₃, CH₂-NR₃R₄ ou phényle,
R₂ représente un atome d'hydrogène ou un groupe alkyle en C₁-C₄,
R₃ représente un atome d'hydrogène ou un groupe alkyle en C₁-C₁₀, linéaire, ramifié ou cyclisé,
R₄ représente un atome d'hydrogène ou un groupe alkyle en C₁-C₁₀ linéaire ou ramifié,
NR₃R₄ pouvant représenter un reste hétérocyclique saturé ayant de 5 à 8 sommets et comportant au moins un atome d'azote,
le carbone porteur du substituant R, lorsque le cycle Q est saturé, peut être de configuration non déterminée (R,S) ou de configuration déterminée (R), ou (S) ; et,
**(ii)** leurs sels d'addition.

2. Composé selon la revendication 1, **caractérisé en ce que** dans la formule I, X est un atome de chlore.

3. Composé selon la revendication 1 **caractérisé en ce que** dans la formule I:
X représente un atome de chlore,
R représente un groupe CO₂-B-R₁ ou CONH-B-R₁, dans lesquels R₁ est CH₂-NR₃R₄, et B est un groupe alkylène en C₁-C₁₀ à chaîne hydrocarbonée linéaire et saturée,
le cycle Q est saturé, et le carbone porteur du substituant R (différent de H) est de configuration déterminée de formule: 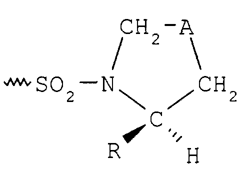
(cette structure correspond à une configuration chirale (S) lorsque A est un groupe CH₂ et à une configuration (R) lorsque A est un atome de soufre).

4. Utilisation d'une substance choisie parmi l'ensemble constitué par les composés de formule I et leurs sels d'addition non toxiques selon la revendication 1 pour l'obtention d'un médicament destiné à une utilisation en thérapeutique vis-à-vis d'états pathologiques impliquant la bradykinine ou ses homologues.

5. Utilisation selon la revendication 4, d'une substance choisie parmi l'ensemble constitué par les composés de formule I et leurs sels d'addition non toxiques selon la revendication 1 pour l'obtention d'un médicament destiné à une utilisation en thérapeutique pour le traitement d'états douloureux.

6. Utilisation selon la revendication 4, d'une substance choisie parmi l'ensemble constitué par les composés de formule I et leurs sels d'addition non toxiques selon la revendication 1 pour l'obtention d'un médicament destiné à une utilisation en thérapeutique pour le traitement d'états inflammatoires.

7. Composition thérapeutique **caractérisée en ce qu'**elle renferme, en association avec un excipient physiologiquement acceptable, au moins un composé de benzènesulfonamide choisi parmi l'ensemble constitué par les composés de formule I et leurs sels d'addition non-toxiques selon la revendication 1.

8. Procédé de préparation d'un composé de formule I selon la revendication 1 où Q est un reste cyclique saturé, et ses sels d'addition, ledit procédé étant **caractérisé en ce qu'**il comprend les étapes consistant à :
**(a)** condenser le composé de formule III : 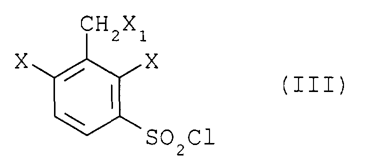 dans laquelle X et X₁ représentent chacun un atome d'halogène,
avec un dérivé hétérocyclique de formule : 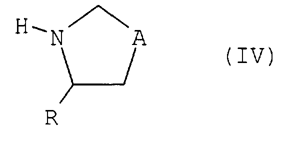 dans laquelle :
A représente un groupe -CH₂-, un groupe -CH(OH)-, un groupe -CH(NH-COCH₃)-, ou un atome de soufre,
R représente un atome d'hydrogène, un groupe COOCH₃ ou un groupe CONHRₐ
dans lequel Rₐ représente un groupe alkyle en C₁-C₃,
le carbone porteur du substituant R étant de configuration (R,S), (R) ou (S)
dans un solvant, en présence d'une base, à une température comprise entre 0 et 40° C, pendant 0,5 à 3 heures, pour obtenir un composé de formule : 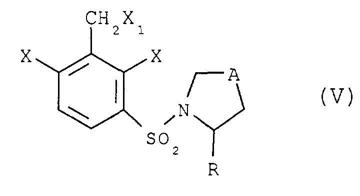 dans laquelle X, A et R conservent la même signification que dans les composés de départ ;
**(b)** faire réagir le composé de formule V ainsi obtenu avec un composé de formule : 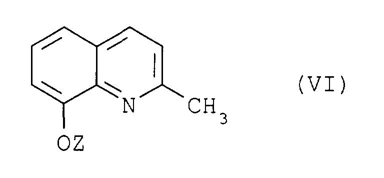 dans lequel Z représente un métal alcalin tel que le sodium ou le potassium,
dans un solvant anhydre, à température comprise entre 0 et 50° C, pendant 0,5 à 5 heures, pour obtenir un composé de formule : 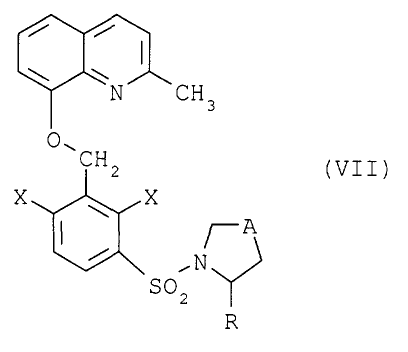 dans laquelle X, A et R conservent la même signification que dans les composés de départ, et le carbone porteur du substituant R conserve la même configuration que dans le composé IV de départ ;
**(c)** si nécessaire, lorsque dans le composé de formule VII ci-dessus R représente un groupe ester, effectuer une hydrolyse alcaline de la liaison ester, dans un solvant, à une température comprise entre 10 et 50° C, pendant 1 à 30 heures pour obtenir, après acidification, le composé acide de formule : 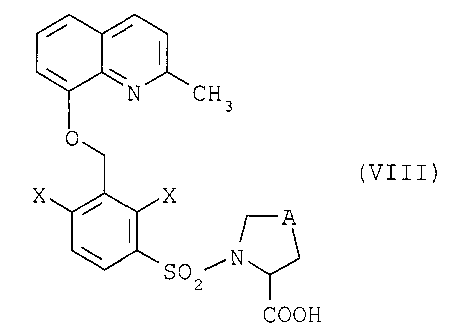 dans laquelle X et A conservent la même signification que dans le composé VII de départ ;
**(d)** si nécessaire, faire réagir l'acide VIII ainsi obtenu avec un alcool ou une amine de formules respectives
HO-B-R₁ (IX) ou HN(R₂)-B-R₁ (IX')
dans lesquelles :
B représente une chaîne alkylène en C₁-C₁₀, linéaire, ramifiée ou comportant un cycle saturé
R₁ représente
un atome d'hydrogène,
un groupe -CH₂-O-CH₃,
un groupe -CH₂-N(CH₃)₂, ou
un groupe phényle,
R₂ représente un atome d'hydrogène ou un groupe alkyle en C₁-C₄,
dans un solvant, en présence d'activateurs, à une température proche de la température ambiante et pendant 2 à 50 heures, pour obtenir un composé de formule : 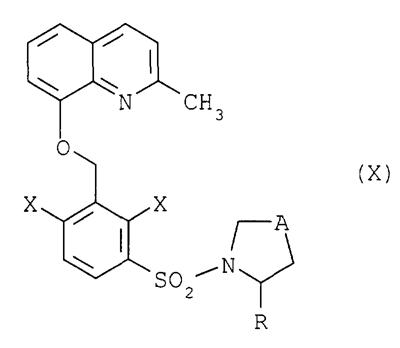 dans laquelle :
X et A conservent la même signification que dans les composés de départ, et
R représente un groupe COO-B-R₁ ou CO-N(R₂)-B-R₁, où B, R₁ et R₂ conservent la même signification que dans les composés IX ou IX' de départ,
le carbone portant le substituant R conservant la même configuration que dans le composé IV de départ.

9. Procédé de préparation d'un composé de formule I selon la revendication 1 où Q est le reste pyrrolidino, et de ses sels d'addition, ledit procédé étant **caractérisé en ce qu'**il comprend la réaction d'un composé de formule : 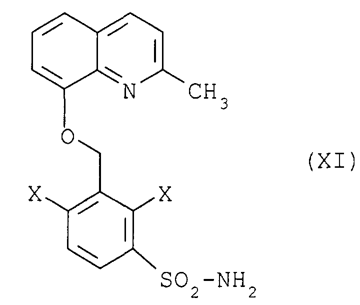 dans laquelle X représente un halogène,
avec du 1,4-dibromobutane, en présence d'une base, dans un solvant, à une température comprise entre 30° C et la température de reflux du solvant, pendant 3 à 30 heures, pour obtenir un composé de formule :

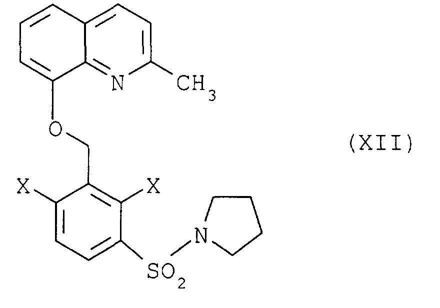

## Patentansprüche

1. Benzolsulfonamidverbindung, **dadurch gekennzeichnet, dass** sie aus der Gesamtheit ausgewählt ist, die von
a) Verbindungen mit der Formel 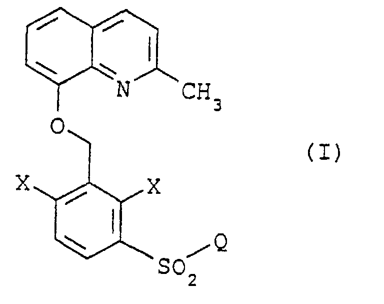 in welcher
X ein Halogenatom,
Q einen N-heterocyclischen Rest mit der Struktur 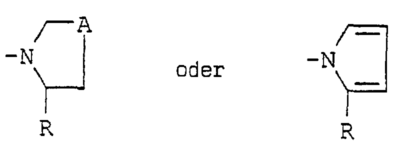
A eine -CH₂-, -CH(OH)- und -CH(NH-COCH₃) -Gruppe oder ein Schwefelatom,
R ein Wasserstoffatom und eine CO₂H-, CO₂-B-R₁- oder CO-N(R₂)-B-R₁-Gruppe,
B eine geradkettige, verzweigte oder cyclische C₁- bis C₁₀-Alkylengruppe,
R₁ ein Wasserstoffatom und eine CH₂OH-, CH₂O-CH₃-, CH₂-NR₃R₄-oder eine Phenyl-Gruppe,
R₂ ein Wasserstoffatom oder eine C₁- bis C₄-Alkylgruppe,
R₃ ein Wasserstoffatom oder eine geradkettige, verzweigte oder cyclische C₁- bis C₁₀-Alkylgruppe und
R4 ein Wasserstoffatom oder eine geradkettige oder verzweigte C₁- bis C₁₀-Alkylgruppe bedeutet und
NR₃R₄ einen gesättigten heterocylischen Rest mit 5 bis 8 Ecken, der mindestens ein Stickstoffatom enthält, bedeuten kann,
wobei das Kohlenstoffträgeratom des Substituenten R, wenn der Cyclus Q gesättigt ist, eine nicht festgelegte Konfiguration (R, S) oder eine festgelegte Konfiguration (R) oder (S) haben kann, und
b) ihren Additionssalzen gebildet wird.

2. Verbindung nach Anspruch 1, **dadurch gekennzeichnet, dass** in der Formel I X ein Chloratom bedeutet.

3. Verbindung nach Anspruch 1, **dadurch gekennzeichnet, dass** in der Formel I
X ein Chloratom und
R eine CO₂-B-R₁- oder CONH-B-R₁-Gruppe, in welcher R₁ CH₂-NR₃R₄ und B eine C₁- bis C₁₀-Alkylengruppe mit gesättigter geradkettiger Kohlenwasserstoffkette bedeutet, bedeutet,
wobei der Cyclus Q gesättigt ist und das Kohlenstoffträgeratom des Substituenten R (der von H verschieden ist) die festgelegte Konfiguration mit der Formel hat: (diese Struktur entspricht einer chiralen Konfiguration (S), wenn A eine CH₂-Gruppe, und einer Konfiguration (R), wenn A ein Schwefelatom bedeutet).

4. Verwendung einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei Erkrankungen vorgesehen ist, an denen Bradykinin oder dessen Homologen beteiligt sind.

5. Verwendung nach Anspruch 4 einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei der Behandlung von Schmerzzuständen vorgesehen ist.

6. Verwendung nach Anspruch 4 einer Substanz, die aus der Gesamtheit ausgewählt ist, die von den Verbindungen mit der Formel I und deren nichttoxischen Additionssalzen nach Anspruch 1 gebildet wird, zur Herstellung eines Arzneimittels, das für eine therapeutische Anwendung bei der Behandlung von Entzündungszuständen vorgesehen ist.

7. Therapeutische Zusammensetzung, **dadurch gekennzeichnet, dass** sie zusammen mit einem physiologisch verträglichen Arzneimittelträger mindestens eine Benzolsulfonamidverbindung enthält, die aus der Gesamtheit ausgewählt ist, die von den Verbindungen mit der Formel I und deren nichttoxischen Additionssalzen nach Anspruch 1 gebildet wird.

8. Verfahren zur Herstellung einer Verbindung mit der Formel I nach Anspruch 1, worin Q einen gesättigten cyclischen Rest bedeutet, und ihren Additionssalzen, wobei das Verfahren
**dadurch gekennzeichnet ist, dass** es die Stufen umfasst, die aus
a) 0,5 bis 3 Stunden langem Umsetzen der Verbindung mit der Formel III 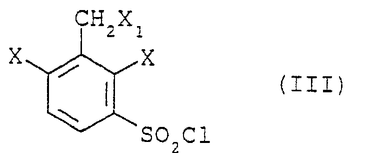 in welcher X und X₁ jeweils ein Halogenatom bedeuten,
mit einer heterocyclischen Verbindung mit der Formel 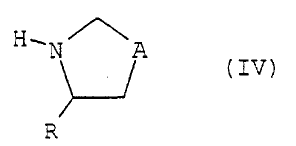 in welcher
A eine -CH₂-, -CH(OH)-, und -CH(NH-COCH₃)-Gruppe oder ein Schwefelatom und
R ein Wasserstoffatom und eine COOCH₃- oder CONHRₐ-Gruppe bedeutet, wobei Rₐ eine C₁- bis C₃-Alkylgruppe bedeutet und das Kohlenstoffträgeratom des Substituenten R die Konfiguration (R,S), (R) oder (S) hat,
in einem Lösungsmittel in Gegenwart einer Base bei einer Temperatur von 0 bis 40 °C, um eine Verbindung mit der Formel 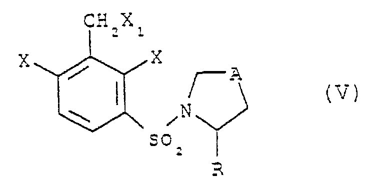 zu erhalten, in welcher X, A und R dieselbe Bedeutung wie in den Ausgangsverbindungen beibehalten,
b) 0,5 bis 5 Stunden langem Umsetzen der so erhaltenen Verbindung mit der Formel V mit einer Verbindung mit der Formel 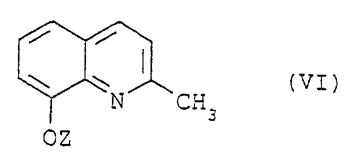 in welcher Z ein Alkalimetall wie Natrium oder Kalium bedeutet, in einem wasserfreien Lösungsmittel bei einer Temperatur von 0 bis 50 °C, um eine Verbindung mit der Formel 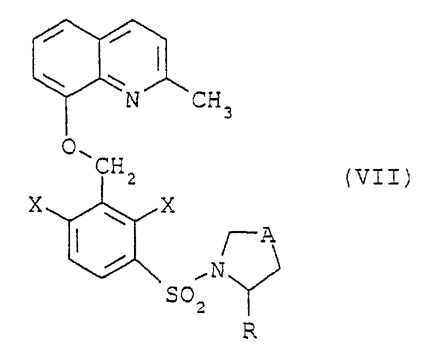 zu erhalten, in welcher X, A und R dieselbe Bedeutung wie in den Ausgangsverbindungen beibehalten und das Kohlenstoffträgeratom des Substituenten R dieselbe Konfiguration wie in der Ausgangsverbindung IV beibehält,
c) erforderlichenfalls, wenn in der Verbindung mit der Formel VII R eine Estergruppe bedeutet, 1 bis 30 Stunden langem Durchführen einer alkalischen Hydrolyse der Esterbindung in einem Lösungsmittel bei einer Temperatur von 10 bis 50 °C, um nach Ansäuern die saure Verbindung mit der Formel 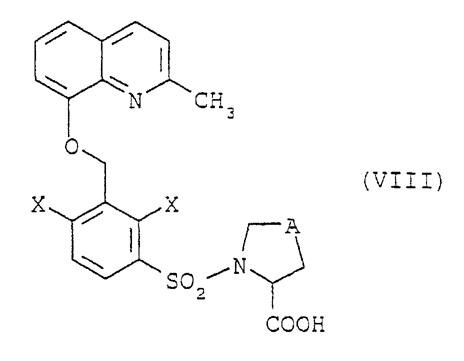 zu erhalten, in welcher X und A dieselbe Bedeutung wie in der Ausgangsverbindung VII beibehalten, und
d) erforderlichenfalls 2 bis 50 Stunden langem Umsetzen der so erhaltenen Säure VIII mit einem Alkohol oder Amin mit der entsprechenden Formel
HO-B-R₁ (IX) bzw. HN(R₂)-B-R₁ (IX'),
in welcher
B eine geradkettige, verzweigte oder einen gesättigten Cyclus enthaltende C₁- bis C₁₀-Alkylenkette,
R₁ ein Wasserstoffatom,
eine -CH₂-O-CH₃-Gruppe,
eine -CH₂-N(CH₃)₂-Gruppe oder
eine Phenylgruppe und
R₂ ein Wasserstoffatom oder eine C₁- bis C₄-Alkylgruppe bedeutet,
in einem Lösungsmittel in Gegenwart von Aktivatoren bei einer Temperatur in der Nähe der Umgebungstemperatur, um eine Verbindung mit der Formel 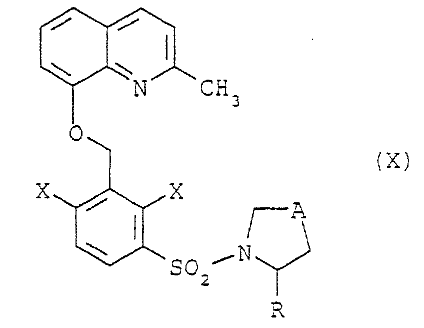 zu erhalten, in welcher
X und A dieselbe Bedeutung wie in den Ausgangsverbindun gen beibehalten und
R eine COO-B-R₁- oder CO-N(R₂)-B-R₁-Gruppe bedeutet, worin B, R₁ und R₂ dieselbe Bedeutung wie in den Ausgangsverbindungen IX bzw. IX' beibehalten, wobei das Kohlenstoffträgeratom des Substituenten R dieselbe Konfiguration wie in der Ausgangsverbindung IV beibehält,
bestehen.

9. Verfahren zur Herstellung einer Verbindung mit der Formel I nach Anspruch 1, worin Q den Pyrrolidinorest bedeutet, und ihren Additionssalzen, wobei das Verfahren **dadurch gekennzeichnet ist, dass** es die 3 bis 30 Stunden lange Umsetzung einer Verbindung mit der Formel 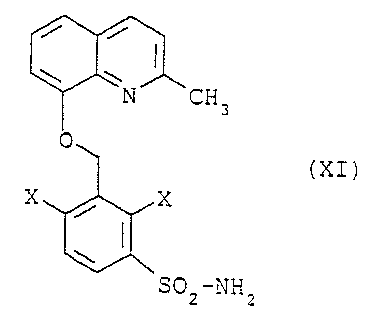 in welcher X ein Halogen bedeutet, mit 1,4-Dibrombutan in Gegenwart einer Base in einem Lösungsmittel bei einer Temperatur von 30 °C bis Rückflußtemperatur des Lösungsmittels umfasst, um eine Verbindung mit der Formel zu erhalten:

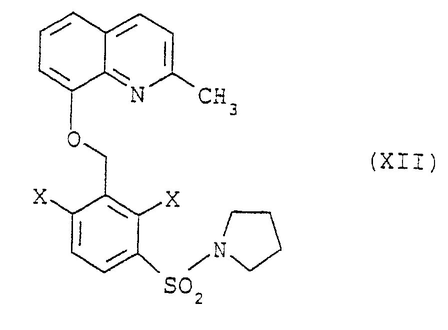

## Claims

1. A benzenesulfonamide compound, **characterized in that** it is selected from the group consisting of:
**(i)** the compounds of the formula 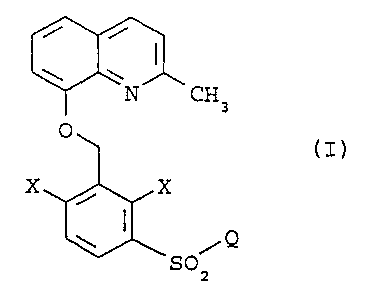 in which:
X is a halogen atom,
Q is an N-heterocyclic radical of the structure 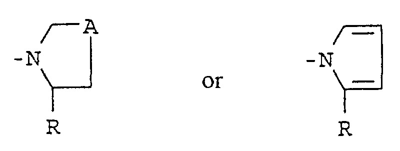
A is a group -CH₂-, -CH(OH)- or -CH(NH-COCH₃)- or a sulfur atom,
R is a hydrogen atom or a group CO₂H, CO₂-B-R₁ or CO-N(R₂)-B-R₁,
B is a linear, branched or cyclic C₁-C₁₀-alkylene group,
R₁ is a hydrogen atom, a group CH₂OH, CH₂-O-CH₃ or CH₂-NR₃R₄ or a phenyl group,
R₂ is a hydrogen atom or a C₁-C₄-alkyl group,
R₃ is a hydrogen atom or a linear, branched or cyclic C₁-C₁₀-alkyl group,
R₄ is a hydrogen atom or a linear or branched C₁-C₁₀-alkyl group,
it being possible for NR₃R₄ to be a saturated heterocyclic radical having from 5 to 8 ring members and containing at least one nitrogen atom, and
the carbon carrying the substituent R, when the ring Q is saturated, can be of indeterminate (R,S) configuration or of determinate (R) or (S) configuration; and
**(ii)** their addition salts.

2. A compound according to claim 1, **characterized in that** X in formula I is a chlorine atom.

3. A compound according to claim 1, **characterized in that** in formula I:
X is a chlorine atom,
R is a group CO₂-B-R₁ or CONH-B-R₁ in which R₁ is CH₂-NR₃R₄ and B is a C₁-C₁₀-alkylene group with a saturated linear hydrocarbon chain,
the ring Q is saturated, and
the carbon carrying the substituent R (other than H) is of determinate configuration of the formula 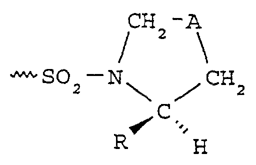 (this structure corresponds to an (S) chiral configuration when A is a group CH₂ and to an (R) configuration when A is a sulfur atom).

4. Use of a substance selected from the group consisting of the compounds of formula I and their non-toxic addition salts according to claim 1 for obtaining a drug intended for use in therapeutics to combat pathological conditions involving bradykinin or its homologs.

5. Use according to claim 4 of a substance selected from the group consisting of the compounds of formula I and their non-toxic addition salts according to claim 1 for obtaining a drug intended for use in therapeutics for the treatment of painful states.

6. Use according to claim 4 of a substance selected from the group consisting of the compounds of formula I and their non-toxic addition salts according to claim 1 for obtaining a drug intended for use in therapeutics for the treatment of inflammatory states.

7. A therapeutic composition, **characterized in that** it contains, in association with a physiologically acceptable excipient, at least one benzenesulfonamide compound selected from the group consisting of the compounds of formula I and their non-toxic addition salts according to claim 1.

8. A process for the preparation of a compound of formula I in which Q is a saturated cyclic radical, and its addition salts, said process being **characterized in that** it comprises the steps which consist in:
**(a)** condensing the compound of formula III: 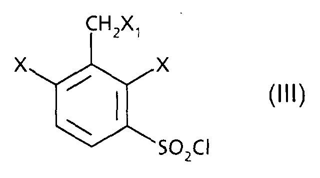 in which X and X₁ are each a halogen atom,
with a heterocyclic derivative of the formula 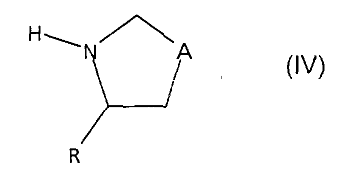 in which:
A is a group -CH₂-, a group -CH(OH)-, a group -CH(NH-COCH₃)- or a sulfur atom, and
R is a hydrogen atom, a group COOCH₃ or a group CONHRₐ, in which Rₐ is a C₁-C₃-alkyl group,
the carbon carrying the substituent R being of (R,S), (R) or (S) configuration,
in a solvent, in the presence of a base, at a temperature between 0 and 40°C, for 0.5 to 3 hours, to give a compound of the formula 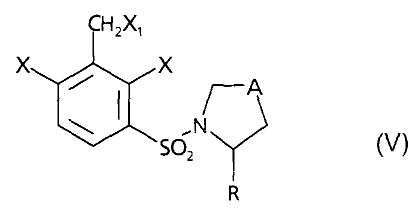 in which X, A and R are as defined in the starting compounds;
**(b)** reacting the resulting compound of formula V with a compound of the formula 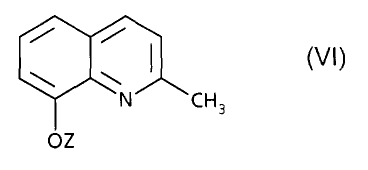 5 in which Z is an alkali metal such as sodium or potassium,
in an anhydrous solvent, at a temperature between 0 and 50°C, for 0.5 to 5 hours, to give a compound of the formula 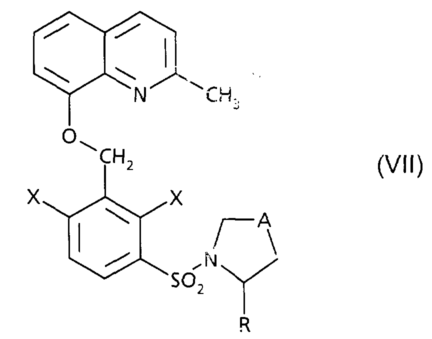 in which X, A and R are as defined in the starting compounds and the carbon carrying the substituent R retains the same configuration as in the starting compound IV;
**(c)** if necessary, when R in the compound of formula VII above is an ester group, carrying out alkaline hydrolysis of the ester linkage in a solvent, at a temperature between 10 and 50°C, for 1 to 30 hours, followed by acidification, to give the acid compound of the formula 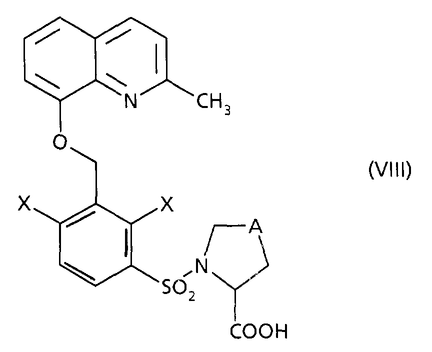 in which X and A are as defined in the starting compound VII; and
**(d)** if necessary, reacting the resulting acid VIII with an alcohol or an amine of the respective formulae
HO-B-R₁ (IX) or HN(R₂)-B-R₁ (IX')
In which:
B is a C₁-C₁₀-alkylene chain which is linear or branched or contains a saturated ring,
R₁ is a hydrogen atom, a group -CH₂-O-CH₃, a group -CH₂-N(CH₃)₂ or a phenyl group, and
R₂ is a hydrogen atom or a C₁-C₄ alkyl group,
in a solvent, in the presence of activators, at a temperature near room temperature, for 2 to 50 hours, to give a compound of the formula 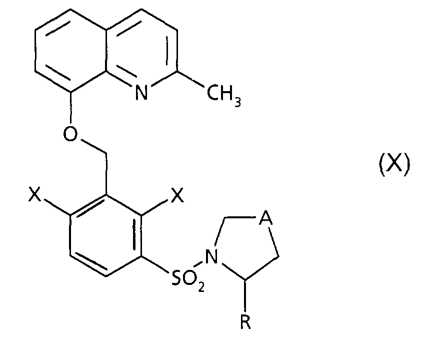 in which:
X and A are as defined in the starting compounds, and
R is a group COO-B-R₁ or CO-N(R₂)-B-R₁ in which B, R₁ and R₂ are as defined in the starting compounds IX or IX',
the carbon carrying the substituent R retaining the same configuration as in the starting compound IV.

9. A process for the preparation of a compound of formula I in which Q is the pyrrolidino radical, and its addition salts, said process being **characterized in that** it comprises reacting a compound of the formula 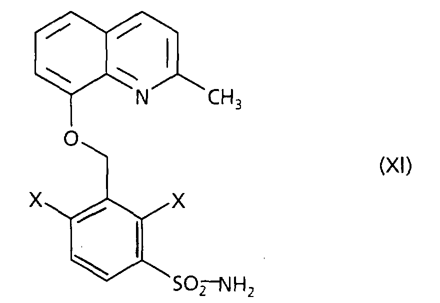 in which X is a halogen,
with 1,4-dibromobutane, in the presence of a base, in a solvent, at a temperature between 30°C and the reflux temperature of the solvent, for 3 to 30 hours, to give a compound of the formula
